# EUROPÄISCHE PATENTANMELDUNG

(11) **EP 2 620 087 A1**
(43) Veröffentlichungstag der Anmeldung: **31.07.2013**
(21) Anmeldenummer: 12401011.7
(22) Anmeldetag: 27.01.2012
(51) Int. Cl.: A47L 15/42

(54) **Geschirrspülmaschine**

(71) Anmelder: Miele & Cie. KG, 33332 Gütersloh (DE)
(72) Erfinder: Abendroth-Pott, Volker, 33611 Bielefeld (DE); Leifeld, Ludger, 59227 Ahlen (DE); Ronning, Christian, 49326 Melle (DE)

(57) **Zusammenfassung**

Die Erfindung betrifft eine Geschirrspülmaschine (1). Um eine Geschirrspülmaschine (1) dahingehend weiterzuentwickeln, dass die Gefahr ungewollter Undichtigkeiten und des damit einhergehenden Sicherheitsrisikos minimiert ist, wird mit der Erfindung vorgeschlagen eine Geschirrspülmaschine (1) mit einem einen Spülraum (3) bereitstellenden Spülbehälter (2), wobei der Spülraum (3) über eine frontseitig des Spülbehälters (2) vorgesehene Spülbehälteröffnung zugänglich ist, die mittels einer um eine horizontal verlaufende Schwenkachse (5) verschwenkbare Gerätetür (4) verschließbar ist, sowie mit einem sich unterhalb des Spülbehälters (2) erstreckenden Sockelbereich (6), der ein der Anordnung von elektronischen Baukomponenten (8) dienendes Gehäuseteil (7) aufnimmt, wobei das Gehäuseteil (7) mit seiner spülbehälterseitigen Randkante (17) unterhalb der Spülbehälteröffnung am Spülbehälter (2) anliegt, wobei zwischen der spülbehälterseitigen Randkante (17) des Gehäuseteils (7) und dem Spülbehälter (2) eine Dichtung (18) angeordnet ist.

## Beschreibung

Die Erfindung betrifft eine Geschirrspülmaschine, insbesondere zu gewerblichen Zwecken in der Ausgestaltung als Reinigungs- und gegebenenfalls Desinfektionsmaschine für medizinische Instrumente und Geräte.

Die Geschirrspülmaschine verfügt über einen einen Spülraum bereitstellenden Spülbehälter, wobei der Spülraum über eine frontseitig des Spülbehälters vorgesehene Spülbehälteröffnung zugänglich ist, die mittels einer um eine horizontal verlaufende Schwenkachse verschwenkbare Gerätetür verschließbar ist. Unterhalb des Spülbehälters erstreckt sich ein Sockelbereich, der ein der Anordnung von elektronischen Baukomponenten dienendes Gehäuseteil aufnimmt.

Geschirrspülmaschinen der eingangs genannten Art sind aus dem Stand der Technik an sich bekannt, weshalb es eines gesonderten druckschriftlichen Nachweises an dieser Stelle nicht bedarf.

Der vom Spülbehälter der Geschirrspülmaschine bereitgestellte Spülraum dient im bestimmungsgemäßen Verwendungsfall der Aufnahme von zu reinigendem Spülgut. Während eines Reinigungsvorganges ist die Spülbehälteröffnung mittels der Gerätetür verschlossen. Dabei erfolgt mittels der Gerätetür ein dem Grunde nach fluiddichter Abschluss, zu welchem Zweck entsprechende Dichtungen vorgesehen sind, die bei geschlossener Tür für einen dichten Abschluss der Spülbehälteröffnung Sorge tragen.

Trotz der zur Abdichtung der Spülbehälteröffnung vorgesehenen Dichtungen kann es im Praxiseinsatz zu ungewollten Undichtigkeiten kommen. Diese können insbesondere dann auftreten, wenn die Dichtungen beschädigt oder verschmutzt sind und ein abdichtendes Anliegen der Dichtung zwischen Spülbehälter und Tür nicht mehr gewährleistet ist. Dabei besteht die Gefahr einer ungewollten Undichtigkeit insbesondere dann, wenn es während eines bestimmungsgemäßen Reinigungsvorganges hinsichtlich der im Spülraum befindlichen Spülflotte zu Aufschäumeffekten kommt. Solche Aufschäumeffekte können insbesondere bei der Reinigung von mit Blut verschmutzten medizinischen Instrumenten und Geräten auftreten.

Infolge einer Aufschäumung der Spülflotte sammelt sich im Spülbehälterunterteil Schaum an, dessen Pegel je nach Aufschäumungsgrad über die untere Randkante der Spülbehälteröffnung hinaus ansteigen kann. Ist die in diesem Bereich zwischen Spülbehälter und Gerätetür vorgesehene Dichtung beschädigt oder verschmutzt, kann es zu einer ungewollten Undichtigkeit und damit zu einem ungewollten Austritt von Spülflotte kommen.

Im schlimmsten Fall kann es infolge einer ungewollten Undichtigkeit zu einem Komplettausfall der gesamten Geschirrspülmaschine kommen. Diese Gefahr besteht insbesondere dann, wenn die ungewollt aus der Geschirrspülmaschine austretende Spülflotte mit elektronischen Baukomponenten in Kontakt kommt, die typischerweise im Sockelbereich unterhalb des Spülbehälters angeordnet sind. Es kann dann zu Kurzschlüssen kommen, die zu einem Komplettausfall der Geschirrspülmaschine führen können. Zudem besteht das Risiko der Brandgefahr.

Ausgehend vom Vorbeschriebenen ist es die **Aufgabe** der Erfindung, eine Geschirrspülmaschine der eingangs genannten Art dahingehend weiterzuentwickeln, dass die Gefahr ungewollter Undichtigkeiten und des damit einhergehenden Sicherheitsrisikos minimiert ist.

Zur **Lösung** dieser Aufgabe wird mit der Erfindung vorgeschlagen eine Geschirrspülmaschine mit einem einen Spülraum bereitstellenden Spülbehälter, wobei der Spülraum über eine frontseitig des Spülbehälters vorgesehene Spülbehälteröffnung zugänglich ist, die mittels einer um eine horizontal verlaufende Schwenkachse verschwenkbare Gerätetür verschließbar ist, sowie mit einem sich unterhalb des Spülbehälters erstreckenden Sockelbereich, der ein der Anordnung von elektronischen Baukomponenten dienendes Gehäuseteil aufnimmt, wobei das Gehäuseteil mit seiner spülbehälterseitigen Randkante unterhalb der Spülbehälteröffnung am Spülbehälter anliegt, wobei zwischen der spülbehälterseitigen Randkante des Gehäuseteils und dem Spülbehälter eine Dichtung angeordnet ist.

Die erfindungsgemäße Geschirrspülmaschine verfügt über ein Gehäuseteil, das vom Sockelbereich unterhalb des Spülbehälters aufgenommen ist. Dieses Gehäuseteil dient der Aufnahme von elektronischen Baukomponenten. Es wird insofern eine gekapselte Anordnung der elektronischen Baukomponenten vorgeschlagen, wobei zum Schutz der elektronischen Baukomponenten ein die elektronischen Baukomponenten aufnehmendes Gehäuseteil vorgesehen ist.

Das Gehäuseteil ist mit einer spülbehälterseitigen Randkante ausgestattet. Diese liegt im endmontierten Zustand unterhalb der Spülbehälteröffnung am Spülbehälter an. Dabei ist in erfindungsgemäßer Weise vorgesehen, dass zwischen der spülbehälterseitigen Randkante des Gehäuseteils einerseits und dem Spülbehälter andererseits eine Dichtung ausgebildet ist. Auf diese Weise wird ein abdichtendes Anliegen des die elektronischen Baukomponenten aufnehmenden Gehäuseteils am Spülbehälter erreicht.

Sollte es im Betriebsfall aufgrund einer verunreinigten oder defekten Türdichtung zu einer ungewollten Leckage, d. h. zu einem ungewollten Austritt von Spülflotte aus dem Spülbehälter kommen, so wird diese nach außen abgeführt. Ein Eindringen der Spülflotte in das die elektronischen Baukomponenten aufnehmende Gehäuseteil ist aufgrund der erfindungsgemäß vorgesehenen Dichtung zwischen Gehäuseteil und Spülbehälter jedenfalls ausgeschlossen. Mit Austritt der Spülflotte nach außen wird die Undichtigkeit für einen Anwender erkennbar, so dass verwenderseitig entsprechende Gegenmaßnahmen sofort ergriffen werden können. Bei aus dem Stand der Technik bekannten Konstruktionen ist eine Undichtigkeit verwenderseitig nicht ohne weiteres erkennbar, weil austretende Spülflotte unbemerkt in den Sockelbereich unterhalb des Spülbehälters gelangen kann.

Bei der zwischen Spülbehälter und Gehäuseteil vorgesehenen Dichtung kann es sich gemäß einem weiteren Merkmal der Erfindung um eine Schaumdichtung handeln. Eine solche Dichtung eignet sich insbesondere deshalb, weil etwaige Fertigungstoleranzen insbesondere hinsichtlich des zumeist aus Kunststoff gebildeten Gehäuseteils ausgeglichen werden können.

Die erfindungsgemäß vorgesehene Dichtung ist gemäß einem weiteren Merkmal der Erfindung als Streifenelement ausgebildet. Dies vereinfacht die Montage. So ist das Streifenelement im Rahmen einer Montage lediglich auf Maß abzulängen und kann alsdann eingesetzt werden. Denkbar ist in diesem Zusammenhang auch die Verwendung von vorkonfektionierten Streifenelementen.

Die Dichtung ist gemäß einem weiteren Merkmal der Erfindung vorzugsweise auf die spülbehälterseitige Randkante des Gehäuseteils aufgeklebt. Dies kann beispielsweise mittels Doppelklebeband erfolgen, wobei auch vorgesehen sein kann, dass die Dichtung randkantenseitig mit einer entsprechenden bereits werksseitig ausgestatteten Klebefläche ausgerüstet ist. Im Montagefall kann die Dichtung nach einer gegebenenfalls vorangegangenen Reinigung der Randkante des Gehäuseteils auf die Randkante durch Kleben aufgebracht werden, so dass eine positionssichere und verrutschfeste Anordnung der Dichtung am Gehäuseteil gewährleistet ist.

Bei Geschirrspülmaschinen der eingangs genannten Art findet eine Bedienung derselben über ein Bedienfeld oder Bedienpult statt. Dieses ist der besseren Handhabung durch einen Verwender türseitig ausgebildet, zumeist im Bereich der oberen Türrandkante. Die elektronischen und elektrischen Baukomponenten der Geschirrspülmaschine sind indes im Sockelbereich unterhalb des Spülbehälters angeordnet. Zum Zwecke der elektrischen und/oder elektronischen Verbindung dieser Baukomponenten mit dem Bedienfeld ist ein Verbindungskabel vorgesehen. Dieses Verbindungskabel kann auch als Kabelbaum bezeichnet werden.

Das zum Zwecke der elektrischen und/oder elektronischen Verbindung vorgesehene Kabel ist typischerweise aus dem Sockelbereich der Maschine heraus in die Gerätetür hineingeführt, innerhalb welcher es zwischen einem Türaußenblech und einem Türinnenblech bis zu dem von der Tür getragenen Bedienfeld geführt ist. Im Falle ungewollter Undichtigkeiten kann aus dem Spülbehälter austretende Spülflotte an dem aus der Gerätetür sockelbereichsseitig austretenden Kabel entlang in den Sockelbereich einströmen. Das notwendigerweise vorzusehende Kabel dient insofern ungewollt als Strömungswegvorgabe für aus dem Spülbehälter austretende Spülflotte.

Um auch diesbezüglich aus Gründen einer verbesserten Dichtigkeit eine Verbesserung vorzuschlagen, ist mit der Erfindung gemäß einem weiteren Merkmal vorgesehen, eine Kabelführung zur bereichsweisen Aufnahme eines die im Gehäuseteil angeordneten elektronischen Baukomponenten mit einem von der Gerätetür getragenen Bedienpult verbindenden Kabels. Erfindungsgemäß ist also eine Kabelführung vorgesehen, die der Führung des Kabels außerhalb der Gerätetür einerseits und des Gehäuseteils andererseits dient.

Dabei verfügt die Kabelführung über einen am Gehäuseteil angeordneten ersten Abschnitt und einen von der Gerätetür getragenen zweiten Abschnitt. Dabei wird im endmontierten Zustand das Kabel von dem Gehäuseteil kommend durch den gehäuseteilseitigen ersten Abschnitt der Kabelführung und von dort aus durch den gerätetürseitigen zweiten Abschnitt der Kabelführung in die Gerätetür hineingeführt.

Zur Kabelführung verfügen die beiden Abschnitte jeweils über einen Kabelaufnahmeraum, wobei sich die beiden Kabelaufnahmeräume jeweils entlang der horizontalen Schwenkachse der Gerätetür erstrecken. Aufgrund dieser Konstruktion ist sichergestellt, dass das von der Kabelführung aufgenommene Kabel im endmontierten Zustand entlang der Schwenkachse der Gerätetür verlegt ist.

Im bestimmungsgemäßen Verwendungsfall verschwenkt der gerätetürseitige zweite Abschnitt der Kabelführung bei einer Verschwenkbewegung der Gerätetür mit. Der gehäuseteilseitige erste Abschnitt der Kabelführung ist hingegen feststehend am Gehäuseteil ausgebildet. Um eine Beschädigung des Kabels beispielsweise durch Bruch infolge einer Torsionsbelastung bei einer Verschwenkung der Gerätetür zu vermeiden, wird mit der Erfindung vorgeschlagen, dass die Kabelaufnahmeräume der beiden Abschnitte der Kabelführung in Längsrichtung der horizontalen Schwenkachse der Gerätetür beabstandet zueinander ausgebildet sind und eine Torsionsstrecke zwischen sich ausbilden. Über die Länge dieser Torsionsstrecke ist das Kabel nicht fest eingespannt, so dass eine tordierende Verdrehbewegung des Kabels bei einem Öffnungs- bzw. Schließvorgang der Gerätetür gestattet ist. Dabei ist die Länge der Torsionsstrecke derart bemessen, dass eine Torsionsbeanspruchung des Kabels vorzugsweise im elastischen Bereich desselben möglich ist. Eine zur Kabelbeschädigung führende Beanspruchung wird so dauerhaft ausgeschlossen.

Zum Schutz des Kabels im Bereich der Torsionsstrecke vor insbesondere mechanischen Krafteinwirkungen ist eine Abdeckung vorgesehen, die sich beispielsweise an einem der beiden Abschnitte der Kabelführung abstützt.

Einer der beiden Abschnitte oder beide Abschnitte der Kabelführung ist bzw. sind mit Austrittsöffnungen zur Abführung von im Undichtigkeitsfall austretender Spülflotte ausgerüstet. Sollte es aufgrund von Undichtigkeiten zu einem Austritt von Spülflotte kommen, so dient das von der Kabelführung aufgenommene Kabel quasi ungewollt als Strömungsbahn. Die erfindungsgemäße Ausgestaltung nutzt diesen Effekt in vorteilhafter Weise aus. So wird im Undichtigkeitsfall austretende Spülflotte entlang des Kabels in die Kabelführung und damit auch in die Abschnitte der Kabelführung eingeleitet. Hier wird das Kabel zumindest bereichsweise horizontal entlang der horizontal verlaufenden Schwenkachse geführt. Am Kabel entlanglaufende Spülflotte tropft der Schwerkraft folgend im Bereich der horizontalen Kabelverlegung nach unten und kann über die Austrittsöffnungen der Kabelführung abfließen. Dabei strömt die abfließende Spülflotte auf die gegenüber dem Spülbehälter abgedichtete Randkante des Gehäuseteils, womit eine zielgerichtete Abführung der Spülflotte nach außen gewährleistet ist. Ein unkontrolliertes Einströmen von Spülflotte in das Gehäuseteil wird so insgesamt wirkungsvoll unterbunden. Darüber hinaus wird ein zielgerichtetes Abführen von ausgetretener Spülflotte erreicht, wobei mit dem Austritt der Spülflotte nach außen zu dem sichergestellt ist, dass ein Verwender der Geschirrspülmaschine möglich zeitnah eine eventuelle Undichtigkeit wahrnehmen kann.

Weitere Merkmale und Vorteile der Erfindung ergeben sich aus der nachfolgenden Beschreibung anhand der Figuren. Dabei zeigen:
- Figur 1: in schematischer und teilgeschnittener Ansicht von vorn eine erfindungsgemäße Geschirrspülmaschine;
- Figur 2: in schematischer Darstellung ausschnittsweise den Sockelbereich einer erfindungsgemäßen Geschirrspülmaschine;
- Figur 3: in einer schematischen Frontansicht ausschnittsweise den Sockelbereich einer erfindungsgemäßen Geschirrspülmaschine;
- Figur 4: in einer schematischen Seitenansicht ausschnittsweise eine erfindungsgemäße Geschirrspülmaschine, und zwar entlang A - A nach Fig. 3 und
- Figur 5: in schematischer Darstellung ausschnittsweise das Gehäuseteil der erfindungsgemäßen Geschirrspülmaschine in Rückansicht.

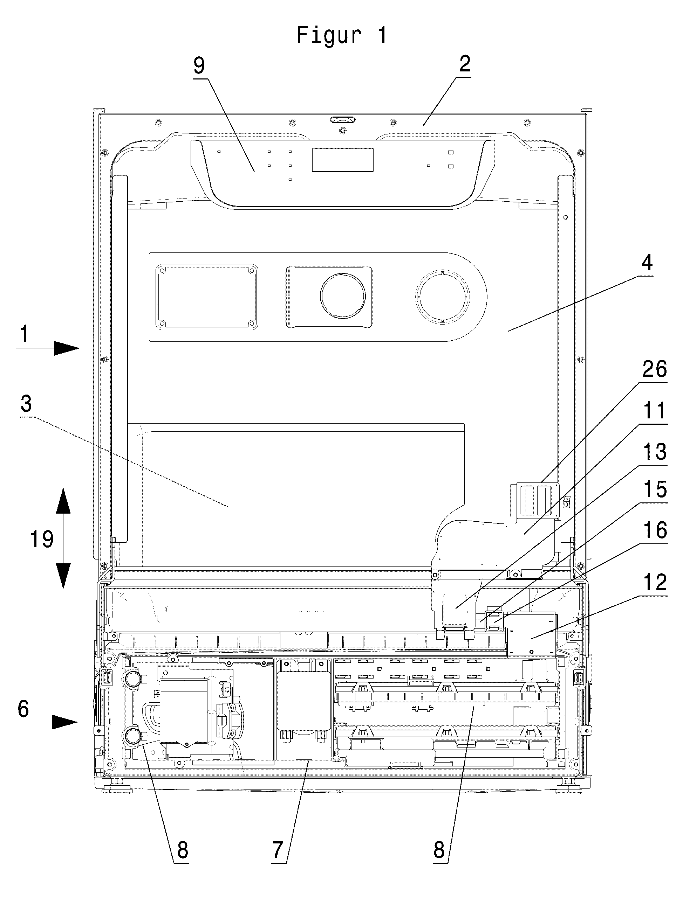

Figur 1 lässt in rein schematischer Ansicht und teilweise geschnitten eine Geschirrspülmaschine 1 nach der Erfindung erkennen. Die Geschirrspülmaschine 1 verfügt über einen Spülbehälter 2, der einen Spülraum 3 bereitstellt. Im bestimmungsgemäßen Verwendungsfall dient der Spülraum 3 der Aufnahme von zu reinigenden Spülgut.

Der Spülraum 3 ist über eine frontseitig des Spülbehälters 2 vorgesehene Spülraumöffnung zugänglich. Diese Spülraumöffnung ist mittels einer um eine horizontal verlaufende Schwenkachse 5 verschwenkbaren Gerätetür 4 verschließbar. Zum Zwecke der Bedienung der Geschirrspülmaschine 1 verfügt diese über ein Bedienpult 9, welches im gezeigten Ausführungsbeispiel an der oberen Randkante der Gerätetür 4 ausgebildet ist.

In Höhenrichtung 19 ist unterhalb des Spülbehälters 2 ein Sockelbereich 6 ausgebildet. Dieser Sockelbereich 6 nimmt ein Gehäuseteil 7 auf. Dieses Gehäuseteil 7 dient der Anordnung von elektronischen Baukomponenten. Auch weitere Baukomponenten, wie z. B. elektrische und/oder mechanische Baukomponenten sind innerhalb des Gehäuseteils 7 angeordnet. Zu den innerhalb des Gehäuseteils 7 angeordneten Baukomponenten gehören beispielsweise der Netzanschluss, die Dosierpumpe, die Hauptelektronik sowie Sicherungshalter.

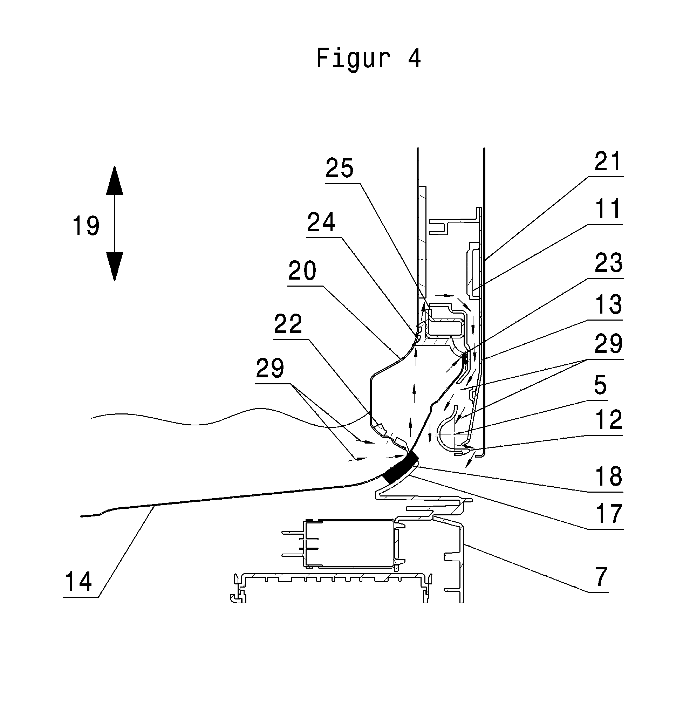

Das Gehäuseteil 7 ist, wie insbesondere die Darstellung nach den Figuren 1 und 4 erkennen lässt, verwenderseitig von vorne, d. h. frontseitig zugänglich. Über eine entsprechend vorgesehene Öffnung können die vom Gehäuseteil 7 aufgenommenen Baukomponenten erreicht werden. Diese Öffnung ist im Betriebsfall durch einen dafür vorgesehenen Deckel verschlossen.

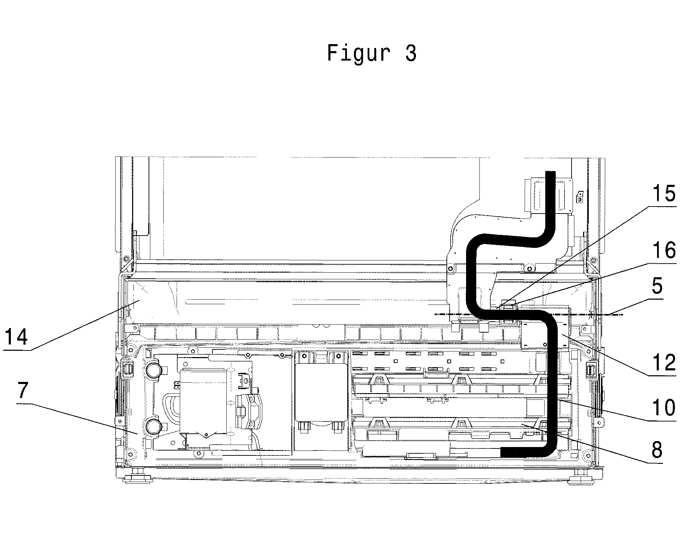

Zur Verbindung der vom Gehäuseteil 7 aufgenommenen elektrischen und/oder elektronischen Baukomponenten 8 mit dem von der Gerätetür 4 getragenen Bedienpult 9 ist ein Kabel 10 vorgesehen, wie sich insbesondere aus der Darstellung nach Figur 3 ergibt.

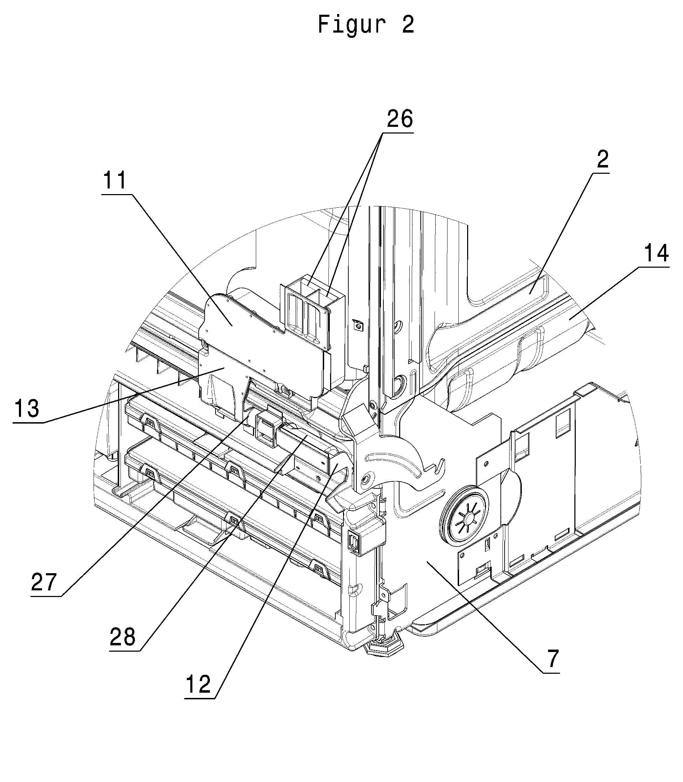

Das Kabel 10 wird aus dem Gehäuseteil 7 in die Gerätetür 4 geführt, wo es im Inneren der Gerätetür 4 bis hin zum Bedienpult 9 verlegt ist. Zum Zwecke der Führung des Kabels 10 im Bereich zwischen Gehäuseteil 7 einerseits und Gerätetür 4 andererseits dient eine Kabelführung 11. Diese ergibt sich insbesondere aus der Ausschnittsdarstellung nach Figur 2.

Die Kabelführung 11 verfügt über einen gehäuseteilseitigen, ersten Abschnitt 12 und einen gerätetürseitigen, zweiten Abschnitt 13 andererseits. Dabei ist der gerätetürseitige, zweite Abschnitt 13 der Kabelführung 11 zusammen mit der Gerätetür 4 verschwenkbar ausgebildet.

Die beiden Abschnitte 12 und 13 stellen jeweils einen Kabelaufnahmeraum bereit, wobei der erste Abschnitt 12 den Aufnahmeraum 28 und der zweite Abschnitt 13 den Aufnahmeraum 27 bereitstellt, wie sich insbesondere aus der Darstellung nach Figur 2 ergibt.

Die beiden Kabelaufnahmeräume 27 und 28 sind in Längsrichtung der horizontalen Schwenkachse 5 der Gerätetür 4 beabstandet zueinander ausgebildet und nehmen zwischen sich eine Torsionsstrecke 15 auf, die mittels einer Abdeckung 16 vor mechanischen Krafteinwirkungen geschützt ausgebildet ist. Die Torsionsstrecke 15 dient im bestimmungsgemäßen Fall der Verschwenkung der Gerätetür 4 dazu, Brüche und/oder Beschädigungen am Kabel 10 durch Torsionsbeanspruchungen zu vermeiden.

Der Spülbehälter 2 verfügt, wie insbesondere die Darstellung nach Figur 4 erkennen lässt, über ein Spülraumunterteil 14. Im endmontierten Zustand liegt das Gehäuseteil 7 mit seiner oberen Randkante 17 am Spülraumunterteil 14 und damit am Spülbehälter 2 an. Dabei ist zwischen der Randkante 17 des Gehäuseteils 7 und dem Spülbehälter 2 eine Dichtung 18 in der bevorzugten Ausgestaltung als Schaumdichtung vorgesehen. Hierdurch wird ein dichtes Anliegen der Randkante 17 des Gehäuseteils 7 am Spülbehälter 2 erreicht.

Die Gerätetür 4 ist doppelwandig ausgestaltete und verfügt über ein Türinnenblech 20 und ein Türaußenteil 21. Das Kabel 10 ist im Bereich der Gerätetür 4 zwischen Türinnenblech 20 und Türaußenteil 21 geführt. Im geschlossenen Zustand der Gerätetür 4, wie er in Figur 4 dargestellt ist, dichtet die Gerätetür 4 gegenüber dem Spülbehälter 2 ab, wobei einerseits eine Türinnenblechdichtung 22 und andererseits eine Dichtung 24 vorgesehen sind, wobei letztere zwischen einem Flachmaterial 25 der Tür 4 und der unteren Randkante der Spülraumöffnung, der Bottichkante 23 ausgebildet ist.

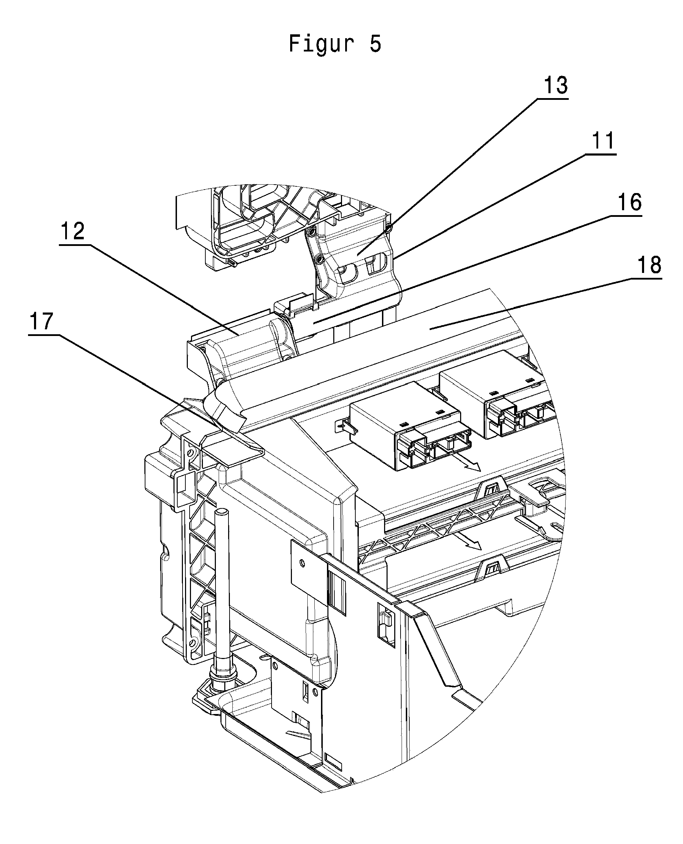

Trotz der zwischen der Gerätetür 4 und dem Spülbehälter 2 vorgesehenen Dichtungen 22 und 24 kann es zu ungewollten Undichtigkeiten kommen. Diese können insbesondere bei beschädigten oder verschmutzten Dichtungen 22 und 24 auftreten. Dabei können solche Undichtigkeiten insbesondere dann auftreten, wenn es im Spülraum 3 zu Aufschäumeffekten der Spülflotte kommt, und zwar mit der Folge, dass entstehende Aufschäumungen im Pegelstand in Höhenrichtung 19 über die untere Bottichkante 23 hinaus innenseitig des Türinnenbleches 20 nach oben aufwandern. In Kombination mit defekten oder verunreinigten Dichtungen 22 und 24 kann es dann zu einem Austritt von Spülflotte kommen. Um ein Eindringen von austretender Spülflotte in die vom Gehäuseteil 7 aufgenommenen Baukomponenten 8 zu unterbinden, ist die schon vorbeschriebene Dichtung 18 zwischen der Randkante 17 des Gehäuseteils 7 und dem Spülbehälter 2 vorgesehen, wie sich insbesondere aus einer Zusammenschau der Figuren 4 und 5 ergibt. Figur 4 lässt zudem anhand von Pfeilen 29 einen möglichen Weg von Spülflotte bei Undichtigkeiten der Dichtungen 22 und 24 erkennen.

Die Kabelführung 11 verfügt gerätetürseitig über eine Kabeldurchführungsöffnung 26, die, wie insbesondere die Darstellung nach Figur 2 erkennen lässt, bei geschlossener Gerätetür 4 oberhalb der Bottichkante 23 zu liegen kommt. Ungewolltes Eindringen von Spülflotte in die Kabelführung 11 durch die Kabeldurchführungsöffnung 26 ist so unterbunden.

Sollte es zu einem ungewollten Spülflottenaustritt kommen, so wird sich diese zumindest zum Teil entlang des Kabels 10 bewegen. Im Bereich der beiden durch die Abschnitte 12 und 13 der Kabelführung 11 bereitgestellten Kabelaufnahmeräume 27 und 28 ist das Kabel 10 horizontal geführt. In diesen Bereichen wird es deshalb der Schwerkraft folgend zu einem Abtropfen von entlang des Kabels 10 strömender Spülflotte kommen. Die Kabelführung 11 verfügt über Austrittsöffnungen, durch die hindurch abtropfende Spülflotte geleitet wird, und zwar auf die dem Spülbehälter 2 zugewandte Randkante 17 des Gehäuseteils 7. Über diese Randkante 17, die als eine Art Rinne fungiert, kann abtropfende Spülflotte nach außen geleitet werden. Dabei ist ein Eindringen von Spülflotte in das Innere des Gehäuseteils 7 aufgrund der zwischen Gehäuseteil 7 und Spülbehälter 2 ausgebildeten Dichtung 18 unterbunden.

### Bezugszeichenliste

- 1: Geschirrspülmaschine
- 2: Spülbehälter
- 3: Spülraum
- 4: Gerätetür
- 5: Schwenkachse
- 6: Sockelbereich
- 7: Gehäuseteil
- 8: Baukomponente
- 9: Bedienpult
- 10: Kabel
- 11: Kabelführung
- 12: erster Abschnitt
- 13: zweiter Abschnitt
- 14: Spülraumunterteil
- 15: Torsionsstrecke
- 16: Abdeckung
- 17: Randkante
- 18: Dichtung
- 19: Höhenrichtung
- 20: Türinnenblech
- 21: Türaußenteil
- 22: Türinnenblechdichtung
- 23: Bottichkante
- 24: Dichtung
- 25: Flachmaterial
- 26: Kabeldurchführungsöffnung
- 27: Kabelaufnahmeraum
- 28: Kabelaufnahmeraum
- 29: Pfeil

## Patentansprüche

1. Geschirrspülmaschine mit einem einen Spülraum (3) bereitstellenden Spülbehälter (2), wobei der Spülraum (3) über eine frontseitig des Spülbehälters (2) vorgesehene Spülbehälteröffnung zugänglich ist, die mittels einer um eine horizontal verlaufende Schwenkachse (5) verschwenkbare Gerätetür (4) verschließbar ist, sowie mit einem sich unterhalb des Spülbehälters (2) erstreckenden Sockelbereich (6), der ein der Anordnung von elektronischen Baukomponenten (8) dienendes Gehäuseteil (7) aufnimmt, wobei das Gehäuseteil (7) mit seiner spülbehälterseitigen Randkante (17) unterhalb der Spülbehälteröffnung am Spülbehälter (2) anliegt, wobei zwischen der spülbehälterseitigen Randkante (17) des Gehäuseteils (7) und dem Spülbehälter (2) eine Dichtung (18) angeordnet ist.

2. Geschirrspülmaschine nach Anspruch 1,
**dadurch gekennzeichnet,**
**dass** die Dichtung (18) eine Schaumdichtung ist.

3. Geschirrspülmaschine nach Anspruch 1 oder 2,
**dadurch gekennzeichnet,**
**dass** die Dichtung (18) als Streifenelement ausgebildet ist.

4. Geschirrspülmaschine nach Anspruch 1, 2 oder 3,
**dadurch gekennzeichnet,**
**dass** die Dichtung (18) auf die spülbehälterseitige Randkante (17) des Gehäuseteils (7) aufgeklebt ist.

5. Geschirrspülmaschine nach einem der vorhergehenden Ansprüche,
**gekennzeichnet durch**
eine Kabelführung (11) zur bereichsweisen Aufnahme eines die im Gehäuseteil (7) angeordneten elektronischen Baukomponenten (8) mit einem von der Gerätetür (4) getragenen Bedienpult (9) verbindenden Kabels (10).

6. Geschirrspülmaschine nach Anspruch 5,
**dadurch gekennzeichnet,**
**dass** die Kabelführung (11) einen am Gehäuseteil (7) angeordneten, ersten Abschnitt (12) und einen von der Gerätetür (4) getragenen, zweiten Abschnitt (13) aufweist.

7. Geschirrspülmaschine nach Anspruch 6,
**dadurch gekennzeichnet,**
**dass** der gerätetürseitige Abschnitt (13) der Kabelführung (11) bedienpultseitig eine Kabeldurchführungsöffnung (26) aufweist, die bei geschlossener Gerätetür (4) oberhalb der unteren Randkante (23) der Spülbehälteröffnung liegt.

8. Geschirrspülmaschine nach Anspruch 6 oder 7,
**dadurch gekennzeichnet,**
**dass** die beiden Abschnitte (12, 13) der Kabelführung (11) jeweils einen Kabelaufnahmeraum (27, 28) bereitstellen, der sich entlang der horizontalen Schwenkachse (5) der Gerätetür (4) erstreckt.

9. Geschirrspülmaschine nach Anspruch 8,
**dadurch gekennzeichnet,**
**dass** die Kabelaufnahmeräume (27, 28) der beiden Abschnitte (12, 13) der Kabelführung (11) in Längsrichtung der horizontalen Schwenkachse (5) der Gerätetür (4) beabstandet zueinander ausgebildet sind und eine Torsionsstrecke (15) zwischen sich ausbilden.

10. Geschirrspülmaschine nach Anspruch 9,
**dadurch gekennzeichnet,**
**dass** die Torsionsstrecke (15) eine Abdeckung (16) trägt, die sich an einem der beiden Abschnitte (12, 13) der Kabelführung (11) abstützt.
